(19) Europäisches Patentamt

European Patent Office

Office européen des brevets

(11) **EP 1 686 182 A1**

(12) **EUROPEAN PATENT APPLICATION**
published in accordance with Art. 158(3) EPC

(43) Date of publication:
   **02.08.2006 Bulletin 2006/31**

(51) Int Cl.:
   *C12P 7/40* (2006.01)    *B01D 9/02* (2006.01)

(21) Application number: **04818203.4**

(22) Date of filing: **05.11.2004**

(86) International application number:
   **PCT/JP2004/016440**

(87) International publication number:
   **WO 2005/045048 (19.05.2005 Gazette 2005/20)**

(84) Designated Contracting States:
   **DE FR**

(30) Priority: **07.11.2003 JP 2003378730**

(71) Applicants:
   • **MITSUBISHI CHEMICAL CORPORATION**
     **Tokyo 108-0014 (JP)**
   • **Ajinomoto Co., Inc.**
     **Tokyo 104-8315 (JP)**

(72) Inventor: **ISOTANI, Atsushi**
   **MITSUBISHI CHEMICAL GROUP SCIENCE**
   **Yokkaichi-shi, Mie 5108530 (JP)**

(74) Representative: **HOFFMANN EITLE**
   **Patent- und Rechtsanwälte**
   **Arabellastrasse 4**
   **81925 München (DE)**

(54) **METHOD FOR PRODUCING ORGANIC ACID SALT**

(57)    A high-purity organic acid salt containing no sugar is produced by heating a mixture of organic acid salt and saccharide, contacting the heated mixture with alcohol, and then separating the organic acid salt from the heated saccharide.

EP 1 686 182 A1

**Description**

Technical Field

The present invention relates to a method for producing a salt of an organic acid having a high melting point such as a dicarboxylic acid, a tricarboxylic acid, or an amino acid. The present invention more specifically relates to a method for producing an organic acid salt from a mixture containing an organic acid salt and saccharide which is obtained from a biological raw material such as glucose or cellulose by microorganism-mediated conversion. Furthermore, the present invention relates to a method for purifying an organic acid salt from a mixture of organic acid salt and saccharide.

Background Art

Organic acids having high melting point such as dicarboxylic acids, tricarboxylic acids, and amino acids are widely used as a raw material for polymers, foods, pharmaceuticals, or the like. For example, succinic acid as a dicarboxylic acid and its derivative are widely used as a raw material for polymers such as polyester and polyamide, and particularly as a raw material for biodegradable polyester or as a raw material for foods, pharmaceuticals, cosmetics, or the like. Furthermore, citric acid as a tricarboxylic acid is widely used as a food additive or the like.

An organic acid such as succinic acid has been industrially obtained so far, by hydrogenation of maleic acid which is a raw material derived from petroleum. However, there have been studied methods of producing organic acids such as succinic acid, malic acid, tartaric acid, and citric acid from a raw material derived from a plant by fermentation using a microorganism.

In the production of an organic acid by fermentation, saccharide is generally used as a raw material, and examples thereof include glucose and cellulose. However, such saccharide may contain as impurities polysaccharide containing maltose as a typical component. The polysaccharide often cannot be consumed by a microorganism and contaminated into an organic acid to be produced. Furthermore, the saccharide as a raw material often cannot be completely consumed by a microorganism and remain to be contaminated into an organic acid. Thus, the produced organic acid and the saccharide should be separated from each other. However, such saccharide has high melting point and is hardly separated from an organic acid by separation utilizing a vapor-liquid equilibrium.

As methods of purifying an organic acid produced by fermentation, there has been known a method using an ion-exchange resin (Patent Document 1), and a method using electrodialysis (Patent Document 2). However, no studies regarding separation of saccharide were conducted in those methods. For example, in the case using a cation-exchange resin, organic acid and saccharide cannot be separated from each other. Furthermore, in the case using electrodialysis, an organic acid is not allowed to pass through, but hydrogen ion as a cation and an alkali metal and ammonia as an anion are allowed to pass through, and thus saccharide and the organic acid cannot be separated. Furthermore, in the case performing crystallization by using water or by directly using a strong acid after treatment with the cation-exchange resin or after electrodialysis, mother liquor which is attached to or accompanied by crystals contains saccharide, and thus a certain level of washing is required for separating the saccharide. However, recrystallization or washing for obtaining a high purity organic acid decreases a recovery ratio of the organic acid. Meanwhile, recycle of an organic acid for improving its recovery ratio has a problem that trace amounts of saccharide accumulate gradually.

In the method using an anion-exchange resin (Patent Document 1), an organic acid is specifically adsorbed, and thus allows separation of the organic acid from saccharide. However, the method has problems that operations for adsorption to the resin, recycle, and the like are complex, and that the resin degrades by repeated recycle operations (Non-patent Document 1).

A separation method using a special device is also known. An example thereof includes a method using a reverse osmosis membrane module from a viewpoint of molecular fractionation (Patent Document 3). Furthermore, in the case of citric acid, there is a method using nano-filtration (Patent Document 4). However, those methods require a specially designed membrane. In particular, the former case (Patent Document 3) aims at rough separation and is not suitable for a purpose of separating saccharide as impurities to purify an organic acid. Another example thereof includes a method of applying a chromatographic separation using a simulated moving bed or the like (Patent Document 5). However, this method requires an additional solvent and an organic acid is diluted several times. Therefore, much energy is consumed for solvent removal, and the cost is too excessive for separating small amounts of saccharide as impurities.

Meanwhile, a method of using crystallized glucose or purified glucose to prevent introduction of a substance which cannot be consumed by bacterial cells or fungi, and a method of prolonging a fermentation time to allow saccharide to be completely consumed have been attempted. Furthermore, in the purification step of an amino acid, or in a conventional purification method using an inorganic acid and the like, a method is applied in which organic acid is recovered as much as possible in one pass without recycling. However, a raw material of high purity is expensive, and complete microorganism-mediated conversion requires a long retention time, that is, a large apparatus. Thus, the method has problems in cost and production efflciency.

In the case that a salt of organic acid is a product, the above-described problems also arise as to the influence of saccharide on quality of the product. For example, in the case that an organic acid salt is obtained as a product by crystallization, crystals contain some mother liquor and saccharide is inevitably contaminated into the organic acid salt. Conventional methods perform washing with water, that is, rinsing, but the organic acid salt has very high water solubility and thus recovery ratio becomes very low. There is a problem of vicious cycle, that is, if a part of a rinsing liquid is recycled for maintaining the recovery ratio, trace amounts of saccharide are accumulated through circulation, which causes further increase in an amount of the rinsing liquid.

[Patent Document 1] US 6,284,904

[Patent Document 2] JP-A-02-283289

[Patent Document 3] JP-A-06-343500

[Patent Document 4] JP-A-10-505840

[Patent Document 5] JP-A-2001-518003

[Non-patent Document 1] Mitsubishi Chemical Corporation Technical Service Series, Diaion, Ion-exchange resin/ synthetic adsorbent manual II

DISCLOSURE OF THE INVENTION

An object of the present invention is to provide a method for producing an organic acid salt of high purity which does not contain saccharide, efficiently and at low cost by a fermentation method using a microorganism. Another object of the present invention is to provide a method for purifying an organic acid salt from a mixture of organic acid salt and saccharide.

The inventor of the present invention has conducted extensive studies for solving the above-mentioned objects. As a result, the inventor of the present invention discovered that saccharide generally insoluble in alcohol undergoes heat denaturation by heating and is converted into a substance which is soluble in methanol, although saccharide has very low solubility in methanol before the heat denaturation. Based on this discovery and a finding that an ammonium salt of organic acid has very low solubility in alcohol and is stable even by heating in the presence of water, the inventor of the present invention has also found that saccharide and ammonium salt of organic acid can be separated from each other by contacting a mixture of the saccharide and the ammonium salt of organic acid with alcohol after heating. Furthermore, the inventor of the present invention also found that other salts of organic acid can also be separated from saccharide in a similar way. Thus, the present invention has been completed.

That is, the present invention is as follows.

(1) A method for producing organic acid salt, which comprises heating a mixture of organic acid salt and saccharide, contacting the heated mixture with alcohol, and then separating the organic acid salt from the heated saccharide.

(2) The method for producing organic acid salt according to (1), wherein said mixture of organic acid salt and saccharide is a mixture which can be obtained by microorganism-mediated conversion of saccharide using a microorganism having an organic acid-producing ability.

(3) The method for producing organic acid salt according to (1) or (2), wherein said mixture of organic acid salt and saccharide is heated at a temperature of not less than 60°C.

(4) The method for producing organic acid salt according to any one of (1) to (3), wherein said organic acid is one or more of organic acids selected from the group consisting of dicarboxylic acids, tricarboxylic acids and amino acids.

(5) The method for producing organic acid salt according to any one of (1) to (3), wherein said organic acid is succinic acid.

(6) The method for producing organic acid salt according to any one of (1) to (5), wherein said saccharide is one or more of saccharide selected from the group consisting of glucose, maltose and sucrose.

(7) The method for producing organic acid salt according to any one of (1) to (6), wherein said alcohol is methanol and the methanol is added at a final concentration of 5 to 60 weight % to be contacted with said mixture.

(8) The method for producing organic acid salt according to any one of (1) to (6), wherein said alcohol is ethanol or propanol and the ethanol or propanol is added at a final concentration of 5 to 60 weight % to be contacted with said mixture in the presence of water of a final concentration of 5 to 50 weight %.

(9) A method for purifying organic acid salt from a mixture of organic acid salt and saccharide, which comprises heating the mixture of organic acid salt and saccharide, contacting the heated mixture with alcohol, and then separating the organic acid salt from the heated saccharide.

(10) A method for producing organic acid salt, which comprises conducting a treatment for solubilizing saccharide against a mixture of organic acid salt and saccharide, then contacting said mixture with a solvent which is a poor solvent for the organic acid salt but can solubilize the saccharide subjected to the treatment for solubilizing saccharide, and separating the organic acid salt.

(11) A method for purifying organic acid salt, which comprises conducting a treatment for solubilizing saccharide against a mixture of organic acid salt and saccharide, then contacting said mixture with a solvent which is a poor solvent for the organic acid salt but can solubilize the saccharide subjected to the treatment for solubilizing saccharide, and separating the organic acid salt.

DESCRIPTION OF THE PREFERRED EMBODIMENTS

Hereinafter, the present invention will be described in more detail.

The production method of the present invention comprises heating a mixture of organic acid salt and saccharide; contacting the heated mixture with alcohol and then separating the organic acid salt from the heated saccharide.

Organic acid which constitutes an organic acid salt to be produced in the present invention is preferably an organic acid that is produced by fermentation using a microorganism, and examples thereof include dicarboxylic acids, tricarboxylic acids, and amino acids. Examples of the dicarboxylic acid include succinic acid, fumaric acid, maleic acid, malic acid, tartaric acid, asparaginic acid, glutaric acid, glutamic acid, adipic acid, suberic acid, itaconic acid, and terephthalic acid. An example of the tricarbocylic acid includes citric acid. Examples of the amino acid include phenylalanine, tryptophan, asparagine, glutamine, valine, isoleucine, leucine, histidine, methionine, and tyrosine. Of those, succinic acid, malic acid, tartaric acid, adipic acid, suberic acid, itaconic acid, glutamic acid, and citric acid are preferable, and succininc acid is particularly preferable.

The salts include sodium salt, potassium salt, ammonium salt, magnesium salt, and calcium salt, and among them, ammonium salt is preferable.

Saccharide contained in the mixture is not limited, but examples thereof include carbohydrates such as galactose, lactose, glucose, maltose, fructose, glycerol, sucrose, saccharose, starch, and cellulose, and fermentable saccharide such as polyalcohol including glycerin, mannitol, xylitol, ribitol, and the like.

The term "mixture of organic acid salt and saccharide" as used in the present invention is not particularly limited, but preferably means a mixture of organic acid salt and saccharide which can be obtained by microorganism-mediated conversion of saccharide using a microorganism having an organic acid-producing ability. The mixture of organic acid salt and saccharide may contain other substances such as medium components.

Hereinafter, methods of obtaining the mixture of organic acid salt and saccharide by culturing a microorganism having an organic acid-producing ability in a medium containing saccharide are described.

The microorganism to be used is a "microorganism having an organic acid-producing ability". The term "microorganism having an organic acid-producing ability" means a microorganism having an ability to produce and accumulate an organic acid in a medium when the microorganism is cultured in a medium containing carbon source as described below. Examples of such a microorganism include facultative anaerobic bacteria such as *Anaerobiospirillum* bacteria (US 5,143,833), *Actinobacillus* bacteria (US 5,504,004), and *Escherichia* bacteria (US 5,770,435), and aerobic bacteria such as coryneform bacteria (JP-A-11-113588) belonging to the genus *Brevibacterium, Corynebacterium, Arthrobacter,* or the like. More preferable examples include a microorganism classified into *Corynebacterium glutamicum, Brevibacterium flavum, Brevibacterium ammoniagenes*, or *Brevibacterium lactofermentum*.

The microorganism to be used may be a microorganism modified so that organic acid-producing ability is enhanced. Examples of such a microorganism include: a microorganism in which expression of a pyruvate carboxylase gene is enhanced (JP-A-11-196888); and a microorganism in which a lactate dehydrogenase gene is disrupted (JP-A-11-206385).

Specific examples of a coryneform bacterium having succinic acid-producing ability include *Brevibacterium flavum* MJ233Δ1dh strain (JP-A-11-206385) in which lactate dehydrogenase activity is decreased, *Brevibacterium flavum* MJ233/pPCPYC strain (WO 01/27258) in which pyruvate carboxylase activity or phosphoenolpyruvate carboxylase activity is enhanced, *Brevibacterium flavum* MJ-233 (FERM BP-1497), *Brevibacterium flavum* MJ-233 AB-41 (FERM BP-1498), *Brevibacterium ammoniagenes* ATCC6872, *Corynebacterium glutamicum* ATCC31831, and *Brevibacterium lactofermentum* ATCC13869.

The microorganism having an organic acid-producing ability to be used in the present invention is not limited to those. Other succinic acid-producing microorganisms, the malic acid-producing microorganisms, fumaric acid-producing microorganisms, citric acid-producing microorganisms, isocitric acid-producing microorganisms, amino acid-producing microorganisms, and the like, which can be obtained by conventional methods may also be used.

A microorganism having an ability to produce two or more kinds of organic acids may also be used.

Saccharide contained in a culture solution is not limited, as long as it can be assimilated by a microorganism. Examples of the saccharide include carbohydrates such as galactose, lactose, glucose, maltose, fructose, glycerol, sucrose, saccharose, starch, and cellulose, and fermentable saccharide such as polyalcohols including glycerin, mannitol, xylitol, ribitol, and the like. Of those, glucose, maltose, and sucrose can be preferably used, and glucose can be particularly preferably used. Furthermore, cellulose that is a main component of paper can also be preferably used as a raw material derived from a plant in a broad sense. Furthermore, a glycated starch liquid, molasses, or the like each containing the above-mentioned fermentable saccharide may also be used. Those carbon sources may be used alone or two or more kinds of them may be used in combination.

A concentration of the saccharide to be used is not particularly limited, but it is advantageous to increase the concentration as high as possible provided that the saccharide does not inhibit the production of organic acid. The saccharide may be used within a concentration range of generally 5 to 30% (W/V), and preferably 10 to 20% (W/V). The saccharide may be supplementary added in accordance with decrease of the saccharide when the reaction progresses.

In addition to the saccharide, the culture solution preferably contains a nitrogen source, an inorganic salt, and the like. The nitrogen source is not particularly limited, as long as it can be assimilated by the microorganism of the present invention to produce a non-amino organic acid. Specific examples thereof include various organic nitrogen compounds and inorganic nitrogen compounds such as ammonium salt, nitrate salt, urea, soybean hydrolysate, casein hydrolysate, peptone, yeast extracts, meat extracts, and corn steep liquor. Phosphate salts, sulfate salts, metal salts such as magnesium salt, potassium salt, manganese salt, iron salt, and zinc salt can be used as an inorganic salt. In addition, to promote the growth of the microorganism, factors such as vitamins (for example, biotin, pantothenic acid, inositol, and nicotinic acid), nucleotides, and amino acids may be added if required. Furthermore, an appropriate amount of a commercially available anti-forming agent is preferably added in advance into a reaction solution to suppress foaming during the reaction.

As the fermentation reaction progresses, an organic acid is produced and pH of the culture solution decreases. Decreased pH causes decrease in metabolic activity of the microorganism or termination of the activity of the microorganism, leading to deterioration of the production rate or cell death of the microorganism. Thus, a neutralizer is preferably used. In general, pH in the reaction system is measured by using a pH sensor, and the pH is adjusted within a predetermined range by addition of a neutralizer. In the present invention, a manner of adding a neutralizer is not particularly limited, and it may be added continuously or intermittently.

Examples of a neutralizer include ammonia, ammonium carbonate, urea, hydroxide of an alkali metal, hydroxide of an alkali earth metal, carbonate salt of alkali metal, and carbonate salt of alkali earth metal. Of those, ammonia, ammonium carbonate, and urea are preferable.

Examples of hydroxide of alkali (earth) metal include NaOH, KOH, Ca(OH)$_2$, and Mg(OH)$_2$, and examples of carbonate salt of alkali (earth) metal include Na$_2$CO$_3$, K$_2$CO$_3$, CaCO$_3$, MgCO$_3$, and NaKCO$_3$. Of those, a neutralizer containing Mg is preferable because such a neutralizer increases the production amount of succinic acid. Two or more kinds of the neutralizers may be used.

pH value of the culture solution is adjusted by a neutralizer within a range in which a microorganism exhibits its activity most effectively, which depends on the kind of a microorganism to be used. The pH value is adjusted within a range of generally 4 to 10, and preferably about 6 to 9.

For production of the mixture of organic acid salt and saccharide by using the above-mentioned microorganism, cells of a microorganism obtained by slant culture on a solid medium such as an agar medium may be used directly, but bacterial cells obtained by culture (seed culture) of the above-mentioned bacterium in advance in a liquid medium is preferably used. In this case, an amount of the bacterial cells to be used in the reaction is not particularly limited, but is generally 1 to 700 g/L, preferably 10 to 500 g/L, and more preferably 20 to 400 g/L.

Culture conditions such as temperature and pressure in the fermentation step vary depending on the microorganism to be used, and conditions suitable for obtaining an organic acid may be selected in each case. For example, the culture temperature is generally 25°C to 40°C, and preferably 30°C to 37°C. The reaction time is preferably 1 hour to 168 hours, and more preferably 3 hours to 72 hours.

After culturing, a mixture containing organic acid or its salt and saccharide accumulates in the culture solution. For example, when the neutralization is performed by using ammonia or urea in the production of an organic acid by microorganism-mediated conversion, the organic acid is obtained as an ammonium salt, and a mixture containing organic acid ammonium salt and saccharide accumulates in a medium. Meanwhile, when the neutralization is performed by using a neutralizer containing alkali metal or alkali earth metal, the organic acid is obtained as a salt of alkali metal or alkali earth metal, and a mixture containing alkali metal salt or alkali earth metal salt of the organic acid and saccharide accumulates in a medium. In this case, the metal salt may be converted into an ammonium salt, to thereby prepare a mixture containing organic acid ammonium salt and saccharide. Such a mixture may be used as the mixture of organic acid salt and saccharide.

In the case of the mixture of organic acid ammonium salt and saccharide, acetic acid may be added to the mixture to precipitate the organic acid; as described below. In this case, a mixture containing ammonium acetate, unreacted organic acid ammonium salt, unreacted acetic acid, and saccharide, which is obtained by separating the precipitated organic acid, may be used as the "mixture of organic acid salt and saccharide". A specific example of the procedure for obtaining the "mixture containing organic acid ammonium salt, acetic acid, ammonium acetate, and saccharide" is shown in the Reference Example described below.

The mixture obtained by fermentation may be isolated from the culture broth by a conventional method after the microbial cells or the like are removed from the culture broth, in which culture has been completed, by centrifugation or the like. Alternatively, the mixture may be a culture filtrate prepared by removing the microbial cells or the like from the broth. In the case that the mixture is used as a culture filtrate, a concentration operation is preferably performed after the removal of the cells. The concentration operation may be performed by a conventional method, and, for example, a kettle reboiler or an evaporator may be used. A multiple-effect evaporator may be used when energy consumption is important for mass production.

In the present invention, the above-mentioned "mixture of organic acid salt and saccharide" is heated and the heated mixture is contacted with alcohol, and then, the organic acid salt is separated from the heated saccharide. The heating is performed at preferably 60 to 180°C, and more preferably at 60 to 160°C. A heating time varies depending on a volume of the mixture, a heat source, and the like, and heating time is preferably 0.1 to 5 hours, and more preferably 0.5 to 3 hours. When a liquid containing the mixture such as a culture filtrate is heated, the mixture is preferably heated while liquid components are concentrated under reduced pressure.

A monovalent to trivalent alcohol is preferably used as alcohol for contacting with the heated mixture, and specific examples thereof include methanol, ethanol, 1-propanol, and 2-propanol. Here, alcohols are exemplified as a solvent to be used for contacting with the heated mixture for separating the organic acid salt from the heated saccharide, however, a solvent which is a poor solvent with respect to the organic acid salt and is capable of dissolving the saccharide subjected to the solubilization treatment can also be used. Methanol as an alcohol is preferably added in an amount so that a final concentration is not less than 5 wt% and not more than 60 wt%.

Ethanol or propanol is preferably added in an amount so that a final concentration is not less than 5 wt% and not more than 60 wt%. In this case, it is preferable that water is also added in the reaction system in an amount providing a final concentration of not less than 5 wt% and not more than 50 wt%. In this case, ethanol or propanol may be added as a water-containing alcohol, that is, as an alcohol containing preferably 4 to 50 wt%, and more preferably 4 to 20 wt% of water.

The step of contacting with alcohol is preferably performed under stirring. The reaction temperature of this step is not particularly limited, but is preferably 0 to 60°C, and more preferably 10 to 40°C. The mixture is preferably contacted with alcohol after the heating step is performed, and then the mixture is cooled to such temperature by leaving to stand. The reaction time varies depending on the amount, composition, and the like of the mixture, but is preferably 0.2 to 4 hours.

By contacting the mixture with alcohol, organic acid in the mixture precipitates because of its low solubility in alcohol, but the saccharide dissolves in the alcohol because the saccharide undergoes heat denaturation under heating to have increased solubility in alcohol. Thus, organic acid salt can be obtained by separation such as filtration. In the present invention, the saccharide subjected to heat denaturation is referred to as "heated saccharide", and heat denaturation treatment by heating is referred to as "solubilization treatment".

The obtained organic acid salt may be used to obtain the organic acid. For this purpose, there can be employed: a method using electrodialysis (JP-A-02-283289); a method using an ion-exchange resin (US 6,284,904 or WO 01/66508); a method of decomposing an organic acid calcium salt, which is produced by fermentation with neutralization with calcium hydroxide, by using sulfuric acid (JP-A-03-030685); a method of performing reactive crystallization by a salt-exchange reaction with sulfuric acid (JP-A-2001-514900 or US 5,958,744); a reactive extraction method (WO 98/01413); and a method using acetic acid (WO 03/95409).

Furthermore, the present invention relates to a method of purifying an organic acid salt from the mixture of organic acid salt and saccharide. The present invention provides the method comprising: heating the mixture; contacting the heated mixture with alcohol; and separating and recovering the organic acid salt from the mixture. The purification method can be performed in the same manner as the corresponding step in the method of producing an organic acid salt as described above.

Hereinafter, a "method of producing an organic acid ammonium salt by using a mixture of an organic acid ammonium salt and saccharide" will be exemplified as one embodiment of the production method of the present invention. However, the method of the present invention is not limited to this embodiment. In the present invention, the term "organic acid ammonium salt" means organic acid monoammonium salt, organic acid diammonium salt, and/or organic acid triammonium salt, unless otherwise noted.

First, a method of obtaining a "mixture of organic acid ammonium salt and saccharide" by microorganism-mediated conversion is explained. A microorganism is cultured in a medium containing saccharide by using one or more kinds of neutralizers selected from the group consisting of ammonia, ammonium carbonate, and urea, to thereby obtain a fermentation solution containing a mixture of organic acid ammonium salt and saccharide.

After completion of the culture, the bacterial cells are separated by centrifugation. The fermentation solution to be obtained is generally a dilute aqueous solution. Direct crystallization of the fermentation solution is difficult, and thus this reaction solution is preferably concentrated. The concentration method is not particularly limited, and examples thereof include evaporation, membrane separation utilizing a reverse osmotic pressure, and electrodialysis using an ion-exchange membrane. Of those, electrodialysis does not have a scale merit and has a problem of high device cost and high operation cost. From the viewpoint of cost and the like, evaporation by heating or the like is preferable, and concentration by using a multiple-effect evaporator is more preferable.

Next, acetic acid is added to the concentrated solution, to thereby precipitate an organic acid (RCOOH) from an organic acid ammonium salt (R-COONH$_4$) according to the reaction formula (I) shown below. Then, the organic acid is separated from mother liquor by filtration. In this case, small amounts of saccharide in the concentrated solution dissolve in acetic acid and remain in the mother liquor (Reference Example described below). Thus, the mother liquor contains unreacted organic acid ammonium salt, excess acetic acid, saccharide, and the produced ammonium acetate. This mother liquor is used as a "mixture containing an organic acid or its salt and saccharide".

$$R\text{-}COONH_4 + CH_3COOH \rightarrow RCOOH\downarrow + CH_3OONH_4 \qquad (I)$$

The above-mentioned mother liquor contains a large volume of solvent, and thus is heated while being concentrated. The concentration operation is performed under heating at 120°C or lower, and preferably 100°C or lower under reduced pressure.

Next, methanol is added to the heated mother liquor to precipitate an organic acid ammonium salt. Then, the precipitated organic acid ammonium salt is separated from the saccharide, acetic acid, and ammonium acetate which are soluble in methanol. This operation may be performed in either of the two ways as follows.

In the first method, just after the heating step, an organic acid ammonium salt is precipitated by adding methanol to the heated mother liquor in the ratio as described above. Crystals to be obtained are organic acid ammonium salt which contains substantially no saccharide. However, in this method, the organic acid ammonium salt to be precipitated and recovered receives no heat history, but recovery rate is lower than that of the second method shown below because of the existence of excess solvent.

The second method is as follows. After the heating step, an ammonium acetate salt is removed as much as possible in the form of acetic acid and ammonia by short-term high temperature treatment (120°C or higher, and preferably 140°C or higher) by using a thin film evaporator or the like. Then, methanol is added to separate the saccharide and obtain the organic acid ammonium salt by precipitation. In this method, an amount of excess solvent is significantly small, and thus a recovery ratio of the organic acid ammonium salt is higher than that of the first method as described above. However, all the organic acid ammonium salt in the mother liquor receives heat history, and thus may be lost through amidation of ammonium ion in the ammonium salt if a retention time in the thin film evaporator or the like is long. Thus, control of the retention time is required.

EXAMPLES

Hereinafter, the present invention will be described in more detail with reference to the examples.

Experimental Example 1: Dissolution test of heat denatured saccharide into methanol

10.01 g of D-glucose (Kishida Chemical Co., Ltd.) and 10.02 g of D-maltose (Kishida Chemical Co., Ltd.) were added into 200cc round-bottom flask and dissolved into 100.04 g of water. The flask was attached to a rotary evaporator and heated at 100°C for 1 hour in oil bath. After heating for 1 hour, no change was observed in particular. Then, the pressure was reduced, and a first distillate fraction was obtained at 340 mmHg. The pressure was further reduced to 30 mmHg for distillation. As a result, a distillate of 92.84 g was obtained, and a paste-like substance remained in the flask. Then, 80.02 g of methanol (Wako Pure Chemical Industries, Ltd.) was added to the round bottom flask and stirred, and thereby the paste-like substance was dissolved completely. It was confirmed that glucose and maltose after heating were dissolved in methanol.

Experimental Example 2: Dissolution test of heat denatured saccharide into methanol (effect of ammonia)

10.01 g of D-glucose (Kishida Chemical Co., Ltd.) and 10.02 g of D-maltose (Kishida Chemical Co., Ltd.) were added into 200cc round-bottom flask and dissolved into 30.32 g of 28% aqueous ammonia (Wako Pure Chemical Industries, Ltd.) and 70.03 g of water. The flask was attached to a rotary evaporator and heated at 100°C for 1 hour in oil bath. After heating for 1 hour, the mixture was colored. Then, the pressure was reduced, and a first distillate fraction was obtained at 460 mmHg. The pressure was further reduced to 30 mmHg for distillation. As a result, a distillate of 83.52 g was obtained, and a brown paste-like substance remained in the flask. Then, 100.16 g of methanol (Wako Pure Chemical Industries, Ltd.) was added to the round bottom flask and stirred, and thereby the paste-like substance was dissolved. The saccharide was converted into a colored substance by heating in the presence of ammonia, and in this case, it was also confirmed that the saccharide after heating was dissolved in methanol.

Experimental Example 3: Dissolution test of heat denatured saccharide into methanol (heating at 80°C)

10.03 g of D-glucose (Kishida Chemical Co., Ltd.) and 10.08 g of D-maltose (Kishida Chemical Co., Ltd.) were added into 200cc round-bottom flask and dissolved into 100.01 g of water. The flask was attached to a rotary evaporator and heated at 80°C for 1 hour in oil bath. After heating for 1 hour; the pressure was reduced, and a first distillate fraction was obtained at 150 mmHg. The pressure was further reduced to 10 mmHg for distillation. As a result, a distillate of 33.25 g was obtained, and a paste-like substance of 27.18 g remained in the flask. There was no material balance because water was not condensed at 10 mmHg and was discharged as a gas. Then, 100.16 g of methanol (Wako Pure Chemical Industries, Ltd.) was added to the round bottom flask and stirred, and thereby the paste-like substance was dissolved completely.

Experimental Example 4: Dissolution test of heat denatured saccharide into methanol (heating at 60°C)

10.00 g of D-glucose (Kishida Chemical Co., Ltd.) and 10.04 g of D-maltose (Kishida Chemical Co., Ltd.) were added into 200cc round-bottom flask and dissolved into 100.09 g of water. The flask was attached to a rotary evaporator and heated at 60°C for 1 hour in oil bath. After heating for 1 hour, the pressure was reduced while the flask was immersed in the 60°C oil bath. The pressure was further reduced to 10 mmHg for distillation. As a result, a distillate of 9.95 g was obtained, and a paste-like substance of 53.87 g remained in the flask. There was no material balance because water was not condensed at 10 mmHg and discharged as a gas. Then, 100.16 g of methanol (Wako Pure Chemical Industries, Ltd.) was added to the round bottom flask and stirred, and thereby the paste-like substance was dissolved completely.
It was revealed that from the Experimental Examples 1 to 4 that the saccharide (glucose and maltose) after heating was dissolved completely into methanol.

Experimental Example 5: Separation of ammonium succinate salt from saccharide

50.02 g of diammonium succinate (Wako Pure Chemical Industries, Ltd.), 10.50 g of D-glucose (Kishida Chemical Co., Ltd.), and 10.01 g of D-maltose (Kishida Chemical Co., Ltd.) were added into 500cc round-bottom flask and dissolved into 150.21 g of water. The flask was attached to a rotary evaporator and heated at 100°C for 1 hour in oil bath. After heating for I hour, the mixture became colored. Then, the pressure was reduced, and a first distillate fraction was obtained at 420 mmHg. The pressure was further reduced to 30 mmHg for distillation. As a result, a distillate of 142.34 g was obtained, and a dried substance and a paste-like substance remained in the flask. Then, 100.07 g of methanol (Wako Pure Chemical Industries, Ltd.) was added into the round bottom flask and stirred, to thereby make the substances into slurry. The slurry was filtered, and thereby, 94.8 g of filtrate and 47.95 g of crystal were obtained. Both substances were analyzed, and it was found that the filtrate contained 7.5 wt% of succinic acid and 1.4 wt% of ammonia, and the crystal contained 63.9 wt% of succinic acid and 14.7 wt% of ammonia.

$$50.02 \text{ g} \times 118/152 = 38.83 \text{ g (introduced succinic acid)}$$

$$47.95 \text{ g} \times 63.9/100 = 30.64 \text{ g (recovered succinic acid)}$$

$$\text{Recovery rate } 30.64/38.83 = 78.9\%$$

In Experimental Example 5, 79% of an organic acid salt (ammonium succinate) was recovered by adding an alcohol under the condition similar to Experimental Examples 1 to 4. The results indicated that there is a surprisingly large difference in solubilities into methanol of the saccharide and the organic acid salt after heating, and that an organic acid salt can be obtained from the mixture of saccharide and organic acid salt by this method.

Experimental Example 6: Dissolution test of heat denatured saccharide into ethanol

10.01 g of D-glucose (Kishida Chemical Co., Ltd.) and 10.02 g of D-maltose (Kishida Chemical Co., Ltd.) were added into 300cc round-bottom flask and dissolved into 101.06 g of water. The flask was attached to a rotary evaporator and heated at 100°C for 1 hour in oil bath.
After heating for 1 hour, no change was observed in particular. Then, the pressure was reduced, and a first distillate fraction was obtained at 380 mmHg. The pressure was further reduced to 25 mmHg for distillation. As a result, a distillate of 78.12 g was obtained, and a paste-like substance remained in the flask. Then, 100.79 g of ethanol (Wako Pure Chemical Industries, Ltd.) was added into the round bottom flask and stirred, but the paste-like substance remained undissolved. Then, water was added thereto in 5 g portions per time, and the paste-like substance was dissolved by addition of water of a total amount of 80 g.

Experimental Example 7: Dissolution test of heat denatured saccharide into 2-propanol

10.01 g of D-glucose (Kishida Chemical Co., Ltd.) and 10.02 g of D-maltose (Kishida Chemical Co., Ltd.) were added into 300cc round-bottom flask and dissolved into 101.06 g of water. The flask was attached to a rotary evaporator and heated at 100°C for 1 hour in oil bath.

After heating for 1 hour, no change was observed in particular. Then, the pressure was reduced, and a first distillate fraction was obtained at 380 mmHg. The pressure was further reduced to 10 mmHg for distillation. As a result, a distillate of 78.12 g was obtained, and a paste-like substance remained in the flask. Then, 100.12 g of 2-propanol (Wako Pure Chemical Industries, Ltd.) was added into the round bottom flask and stirred, but the paste-like substance remained undissolved. Then, water was added thereto in 5 g portions per time, and the paste-like substance was dissolved by addition of water of a total amount of 60 g.

Experimental Example 8: Separation of sodium citrate from saccharide by using 2-propanol

10.04 g of D-glucose (Kishida Chemical Co., Ltd.), 10.01 g of D-maltose (Kishida Chemical Co., Ltd.), and 50.08 g of sodium citrate were added into 300cc round-bottom flask and dissolved into 100.10 g of water. The flask was attached to a rotary evaporator and heated at 100°C for 1 hour in oil bath. After heating for 1 hour, the mixture became yellowish. Then, the pressure was reduced, and a first distillate fraction was obtained at 3 80 mmHg. The pressure was further reduced to 10 mmHg for distillation. As a result, a distillate of 75.63 g was obtained. 60.01 g of water was added into the round bottom flask and stirred, and thereby the distillate was gradually dissolved over time. Then, 100.0 g of 2-propanol (Wako Pure Chemical Industries, Ltd.) was added, and thereby white crystal was precipitated. The precipitate was filtered, to yield 57.33 g of the white crystal. The white crystal was analyzed and found to contain 38.1 wt% of citric acid and 17.2 wt% of sodium.

It was revealed from Experimental Examples 6 and 7 that the saccharide (glucose and maltose) after heating was completely dissolved into water-containing ethanol or water-containing 2-propanol. In Experimental Example 8, the organic acid salt (sodium citrate) was recovered by adding alcohol under the condition similar to Experimental Examples 6 and 7. The results indicated that an organic acid salt can be obtained from the mixture of saccharide and the organic acid salt by separating the saccharide from the mixture.

Experimental Example 9: Dissolution test of heat denatured saccharide into ethanol

5.05 g of D-glucose (Kishida Chemical Co., Ltd.), 5.01 g of D-maltose (Kishida Chemical Co., Ltd.), 100.0 g of water, and 30.02 g of ammonium acetate were added into a simple distillation device equipped with 500cc flask, and dissolved. The flask was heated at normal pressure for 1 hour in 100°C oil bath. The temperature inside the flask was 90°C. After heating for 1 hour, the mixture became brown. The pressure was reduced to 10 mmHg, and a vapor was distilled off. A distillate of 52.84 g was obtained. The amount of the residual substance in the flask was calculated by subtracting tare, resulting in a net weight of 19.43 g. The substance was in a paste-like state in the flask. Because of a high degree of vacuum, much of the distillate presumably was not condensed by the condenser and discharged as a gas. 100.1 g of ethanol (Wako Pure Chemical Industries, Ltd.) was added into the flask. The mixture was not completely uniform, and 16 g of water was further added thereto and stirred, and thereby the paste-like substance was dissolved completely. It was confirmed that glucose and maltose after heating were dissolved in ethanol containing 15% of water.

Experimental Example 10: Separation of ammonium succinate from saccharide by using ethanol

5.22 g of D-glucose (Kishida Chemical Co., Ltd.), 5.19 g of D-maltose (Kishida Chemical Co., Ltd.), 100.03 g of water, and 30.03 g of ammonium succinate were added into a simple distillation device equipped with 500cc flask, and dissolved. The flask was heated at normal pressure for I hour in 100°C oil bath. The temperature inside the flask was 90°C. After heating for 1 hour, the mixture became brown. The pressure was reduced to 16 mmHg, and a vapor was distilled off. A distillate of 33 g was obtained. The amount of the residual substance in the flask was calculated by subtracting tare, resulting in a net weight of 50.48 g. The substance was in a paste-like state in the flask. Then, 100.1 g of ethanol (Wako Pure Chemical Industries, Ltd.) and 20 g of water were added into the flask. The mixture was not completely uniform. The mixture was stirred sufficiently and then filtered.

As a result, 18.68 g of a solid content and 120.55 g of a filtrate were obtained. The filtrate was left to stand for not less than 1 hour, and it was observed that the filtrate was separated into two layers of a light brown solution and a dark brown solution.

On the other hand, the solid content had a composition of 68.6 wt% of succinic acid and 17.6% of ammonia, and it was confirmed that succinate was separated from the saccharide.

It was revealed from Experimental Example 9that the saccharide (glucose and maltose) after heating were completely dissolved into water-containing ethanol. In Experimental Example 10, an organic acid salt (ammonium succinate) was recovered by adding water-containing ethanol under the condition similar to Experimental Example 9. The results indicated that an organic acid salt can be obtained from the mixture of saccharide and organic acid salt by separating the saccharide from the mixture.

Table 1 collectively shows the conditions and results of the Experimental Examples 1 to 10.

## Table 1

Table. 1

| | | Experiment 1 | Experiment 3 | Experiment 4 | Experiment 5 | Experiment 6 | Experiment 9 | Experiment 10 | Experiment 7 | Experiment 8 |
|---|---|---|---|---|---|---|---|---|---|---|
| | | Standard condition | Reference | Minimum heating temperature | Separation from succinate salt | Ethanol | Ethanol (ammonia) | Separation from succinate salt | 2-Propanol | Separation from citrate salt |
| **Added amounts** | | | | | | | | | | |
| Glucose | g | 10.06 | 10.03 | 10.00 | 10.50 | 10.01 | 5.05 | 5.22 | 10.02 | 10.04 |
| Maltose | g | 10.03 | 10.08 | 10.04 | 10.01 | 10.02 | 5.01 | 5.19 | 10.01 | 10.01 |
| Water | g | 100.04 | 100.01 | 100.09 | 150.21 | 101.06 | 100 | 100.03 | 100.03 | 100.1 |
| Aqueous ammonium (28%) | g | | | | | | | | | |
| Ammonium acetate | g | | | | | | 30.02 | | | |
| Ammonium succinate | g | | | | 50.02 | | | | | |
| Citrate 3Na/2hydrate | g | | | | | | | | | 50.08 |
| Total | g | 120.13 | 120.12 | 120.13 | 220.74 | 121.09 | 140.08 | 140.47 | 120.06 | 170.23 |
| **Heating process** | | | | | | | | | | |
| Heating temperature | °C | 100 | 80 | 60 | 100 | 100 | 100 | 100 | 100 | 100 |
| Heating time | hr | 1 | 1 | 1 | 1 | 1 | 1 | 1 | 1 | 1 |
| Remarks | | colorless | colorless | colorless | colored | colorless | colored | colored | colorless | colored |
| Distilled amount | g | 92.84 | 92.94 | 66.26 | 142.34 | 78.12 | 52.84 | 33 | 73.71 | 75.63 |
| Final pressure of distillation | mmHg | 30 | 10 | 10 | 30 | | 10 | 16 | 10 | 10 |
| Residual amount in flask | g | 27.29 | 27.18 | 53.87 | 78.4 | 42.97 | 19.43 | 50.48 | 21.41 | 89.3 |
| State in flask after distillation | | liquid state | liquid state | liquid state | partially precipitated | | paste | partially precipitated as paste | | |
| **Added alcohol** | | | | | | | | | | |
| Methanol | g | 80.02 | 100.16 | 100.16 | 100.07 | | | | | |
| Ethanol | g | | | | | 100.79 | 100.1 | 100.1 | | |
| 2-Propanol | g | | | | | | | | 100.12 | 100 |
| Water | g | | | | | 80 | 16 | 20 | 60 | 60.01 |
| Weight% of alcohol | wt% | 74.6% | 78.7% | 65.0% | 56.1% | 45.0% | 73.9% | 58.7% | 55.2% | 40.1% |
| Operation for separation of crystal | | dissolved | dissolved | dissolved | slurry | dissolved | dissolved | some precipitates, and separated into three phases of liquid-liquid-solid | dissolved | dissolved by addition of water, precipitated by addition of propanol |
| Collected crystal | g | 0 | 0 | 0 | 47.95 | 0 | 0 | 18.68 | 0 | 57.33 |
| Filtrate | g | | | | 94.8 | | | 120.55 | | 170.2 |
| Organic acid in crystal | wt% | | | | 63.9 | | | 68.6 | | 38.1 |
| | | | | | Succinate | | | Succinate | | Citrate |
| Recovery rate of succinate | | | | | 78.90% | | | 54.97% | | 66.74% |
| Comments | | Soluble in methanol | Soluble in methanol | Soluble in methanol | Succinate was precipitated, and color was confirmed by Maillard reaction | Water is required when ethanol is used | Solubility is enhanced in the presence of ammonia | Succinate salt was precipitated, and considered to be colored based on the reaction | Water is required when propanol is used | Citrate salt was precipitated, and considered to be colored based on the reaction |

Reference Example

Crystallization of ammonium succinate with acetic acid

180.28 g of diammonium succinate, 119.75 g of acetic acid, 139.10 g of ion-exchanged water, 20.12 g of ammonium acetate, and 5.0 g of glucose were added into a crystallization device, dissolved at 100°C, and then cooled to 30°C for crystallization. 1.02 g of succinic acid as a seed crystal was added to the mixture. 5 minutes after the addition of the seed crystal, precipitation started. After stirring of the mixture for 40 minutes, suction filtration was started. After filtration for 1 hour 15 minutes, solid contents were taken out. As a result, 302.44 g of a filtrate and 145.5 g of a solid content were obtained. The obtained solid content was analyzed, resulting in 59.0 wt% of succinic acid and 14.6 wt% of acetic acid, with no glucose. The obtained mother liquor was analyzed, resulting in 18.1 wt% of succinic acid, 36.3 wt% of acetic acid, and 1.3 wt% of glucose.

According to the Reference Example, glucose is dissolved into monocarboxylic acid and concentrated in mother liquor after reactive crystallization. Thus, the mother liquor containing ammonium succinate and saccharide can be used as the "mixture containing organic acid salt and saccharide" for performing the production method of the present invention.

INDUSTRIAL APPLICABILITY

The method of the present invention allows production of organic acid salt of high purity which does not contain a mixture of saccharide efficiently and at low cost by a fermentation method using a microorganism. Furthermore, the method of the present invention allows purification of organic acid salt from the mixture of the organic acid salt and saccharide.

**Claims**

1. A method for producing organic acid salt, which comprises heating a mixture of organic acid salt and saccharide, contacting the heated mixture with alcohol, and then separating the organic acid salt from the heated saccharide.

2. The method for producing organic acid salt according to claim 1, wherein said mixture of organic acid salt and saccharide is a mixture which can be obtained by microorganism-mediated conversion of saccharide using a microorganism having an organic acid-producing ability.

3. The method for producing organic acid salt according to claim 1 or 2, wherein said mixture of organic acid salt and saccharide is heated at a temperature of not less than 60°C.

4. The method for producing organic acid salt according to any one of claims 1 to 3, wherein said organic acid is one or more of organic acids selected from the group consisting of dicarboxylic acids, tricarboxylic acids and amino acids.

5. The method for producing organic acid salt according to any one of claims 1 to 3, wherein said organic acid is succinic acid.

6. The method for producing organic acid salt according to any one of claims 1 to 5, wherein said saccharide is one or more of saccharide selected from the group consisting of glucose, maltose and sucrose.

7. The method for producing organic acid salt according to any one of claims 1 to 6, wherein said alcohol is methanol and the methanol is added at a final concentration of 5 to 60 weight % to be contacted with said mixture.

8. The method for producing organic acid salt according to any one of claims 1 to 6, wherein said alcohol is ethanol or propanol and the ethanol or propanol is added at a final concentration of 5 to 60 weight % to be contacted with said mixture in the presence of water of a final concentration of 5 to 50 weight %.

9. A method for purifying organic acid salt from a mixture of organic acid salt and saccharide, which comprises heating the mixture of organic acid salt and saccharide, contacting the heated mixture with alcohol, and then separating the organic acid salt from the heated saccharide.

10. A method for producing organic acid salt, which comprises conducting a treatment for solubilizing saccharide against a mixture of organic acid salt and saccharide, then contacting said mixture with a solvent which is a poor solvent for the organic acid salt but can solubilize the saccharide subjected to the treatment for solubilizing saccharide, and separating the organic acid salt.

**11.** A method for purifying organic acid salt, which comprises conducting a treatment for solubilizing saccharide against a mixture of organic acid salt and saccharide, then contacting said mixture with a solvent which is a poor solvent for the organic acid salt but can solubilize the saccharide subjected to the treatment for solubilizing saccharide, and separating the organic acid salt.

**EP 1 686 182 A1**

## INTERNATIONAL SEARCH REPORT

International application No.

PCT/JP2004/016440

A. CLASSIFICATION OF SUBJECT MATTER
Int.Cl⁷ C12P7/40, B01D9/02

According to International Patent Classification (IPC) or to both national classification and IPC

B. FIELDS SEARCHED

Minimum documentation searched (classification system followed by classification symbols)
Int.Cl⁷ C12P7/40, B01D9/02

Documentation searched other than minimum documentation to the extent that such documents are included in the fields searched

Electronic data base consulted during the international search (name of data base and, where practicable, search terms used)
CAPLUS/MEDLINE/BIOSIS/WPIDS(STN)

C. DOCUMENTS CONSIDERED TO BE RELEVANT

| Category* | Citation of document, with indication, where appropriate, of the relevant passages | Relevant to claim No. |
|---|---|---|
| X | WO 2003/033448 A1 (BASF AG.), 24 April, 2003 (24.04.03), Claims; example 1 & DE 10149869 A1          & EP 1436245 A1 & AU 2002350511 A1       & US 2004/262161 A1 | 1-11 |
| A | WO 2001/09074 A1 (ARCHER DANIELS MIDLAND CO.), 08 February, 2001 (08.02.01), Particularly, example 4 & AU 7390400 A          & BR 0012953 A & CA 2380168 A          & EP 1206435 A1 & US 2002/055155 A1      & JP 2003-518366 A | 1-11 |

☐ Further documents are listed in the continuation of Box C.  ☐ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 26 January, 2005 (26.01.05) | 15 February, 2005 (15.02.05) |

| Name and mailing address of the ISA/ | Authorized officer |
|---|---|
| Japanese Patent Office | |
| Facsimile No. | Telephone No. |

Form PCT/ISA/210 (second sheet) (January 2004)

14